Europäisches Patentamt

(19) European Patent Office

Office européen des brevets

(11) Numéro de publication : **0 317 406 B1**

(12) **FASCICULE DE BREVET EUROPEEN**

(45) Date de publication du fascicule du brevet :
**29.01.92 Bulletin 92/05**

(21) Numéro de dépôt : **88402836.6**

(22) Date de dépôt : **10.11.88**

(51) Int. Cl.⁵ : **A61F 2/08**

(54) **Cheville artificielle par la fixation intra-osseuse de prothèses ligamentaires et renforts ligamentaires, ainsi que l'impacteur associé comportant des moyens de maintien de ladite cheville.**

(30) Priorité : **16.11.87 FR 8715787**

(43) Date de publication de la demande :
**24.05.89 Bulletin 89/21**

(45) Mention de la délivrance du brevet :
**29.01.92 Bulletin 92/05**

(84) Etats contractants désignés :
**BE DE ES GB IT**

(56) Documents cités :
**EP-A- 0 238 223**
**EP-A- 0 241 792**
**DE-A- 2 818 254**

(73) Titulaire : **Laboureau, Jacques-Philippe**
**24 rue de la Fontaine Billenois**
**F-21000 Dijon (FR)**

(72) Inventeur : **Laboureau, Jacques-Philippe**
**24 rue de la Fontaine Billenois**
**F-21000 Dijon (FR)**

(74) Mandataire : **Bruder, Michel**
**Cabinet Michel Bruder Conseil en Brevets 10,**
**rue de la Pépinière**
**F-75008 Paris (FR)**

## Description

La présente invention concerne un ensemble formé d'une cheville conique artificielle pour la fixation intraosseuse de prothèses ligamentaires et renforts ligamentaires ainsi que l'impacteur associé comportant des moyens de maintien de ladite cheville rendant l'ensemble particulièrement adapté à la chirurgie sous arthroscopie. Un tel ensemble est déjà connu du document EP 241792 pour la fixation intraosseuse de prothèses.

La fixation de ligaments artificiels à l'intérieur d'articulation de genou était jusqu'à maintenant obtenue au moyen d'une cheville osseuse confectionnée artisanalement au ciseau à partir de fragment d'os prélevé sur la face antérieure du tibia ; cette technique présente d'ailleurs des avantages considérables en ce qu'elle utilise d'abord une cheville d'excellente qualité, en ce qu'elle évite ensuite des contrincisions habituellement nécessaires pour fixer le ligament sur la partie extérieure de l'os.

En revanche de nombreuses difficultés ressortent de l'utilisation d'une cheville osseuse et notamment les effets nocifs de rallonger les opérations, de prélever et façonner un morceau d'os, de faire des hématomes etc. ; pour remédier en grande partie à ces inconvénients on a pensé utiliser une cheville artificielle en remplacement de la cheville osseuse notamment pour une implantation par voie andoarticulaire évitant de la sorte toute contrincision ; un premier avantage important de cette nouvelle cheville artificielle est d'être utilisable partout et donc aussi par voie extra-articulaire ; réalisable en plusieurs tailles dans un matériau biocompatible suivant des formes coniques régulières, cette cheville, accouplée à un impacteur de forme adéquate constituant un ensemble rigide facile à diriger et particulièrement adapté aux opérations effectuées sous arthroscopie, est caractérisée en ce que ladite cheville comporte, extérieurement, en partie inférieure de sa surface latérale des éléments anti-retour dirigés vers la base de la cheville, cette base étant par ailleurs constituée d'une calotte sensiblement hémisphérique, en intérieurement suivant son axe longitudinal un insert de diamètre adéquat, disposé dans un puits débouchant dans le chambrage de fixation de l'impacteur prévu à la base de la cheville, et s'étendant sur une longueur supérieure à la partie de cheville comportant les éléments anti-retour.

Par sa forme conique régulière et ses éléments anti-retours, la cheville artificielle suivant l'invention apporte un surcroît de sécurité dans le blocage mécanique des ligaments artificiels par rapport aux chevilles osseuses de forme naturellement irrégulières ; on a d'ailleurs pu mettre en évidence que le blocage mécanique ainsi obtenu procure une résistance à l'arrachement largement supérieure à la force de rupture des ligaments ; ceci constitue un avantage décisif en cas de reprise précoce des activités sportives chez les sujets nouvellement implantés de ligaments artificiels.

Une autre caractéristique de la cheville artificielle tient à sa base en forme de calotte hémisphérique qui vient protéger le ligament artificiel du rebord tranchant du tunnel osseux dans lequel il est implanté, lors des mouvements d'extension de la jambe.

Il est en effet habituel de positionner la cheville autour du ligament croisé antérieur du genou notamment, en une zone située entre "12 heures" et "3 heures" dans le condyle externe de l'os ; dans ces conditions le ligament vient, dans les mouvements d'extention de la jambe, s'enrouler autour de la base hémisphérique de la cheville artificielle qui l'éloigne ainsi du bord tranchant du tunnel osseux et réduit de ce fait considérablement les risques de rupture de ligament artificiel.

Suivant une autre caractéristique principale de l'invention un insert métallique est prévu pour renforcer axialement la cheville artificielle, du moins sur toute sa partie affaiblie par le système anti-retour qui est obtenu par enlèvement de matière à partir de la cheville conique lisse, de façon à éviter tout élément débordant de l'enveloppe de la cheville qui nuirait à la précision de positionnement ou encore à la régularité de pénétration de la cheville dans le tunnel d'implantation.

Un avantage tout à fait inattendu ressort aussi de l'inclusion d'un insert métallique radio-opaque dans l'âme de la cheville artificielle en ce qu'il permet son repérage par radiographie ; il est en effet rassurant pour le chirurgien pratiquant des implantations de ce type de pouvoir isoler instantanément toute cheville artificielle qui, en l'espèce, aurait été arrachée accidentellement.

Enfin, dans une dernière caractéristique de l'invention, il a été étudié un matériel ancillaire du type impacteur pour le positionnement et l'introduction de la cheville artificielle dans le tunnel osseux pratiqué dans l'articulation par la voie intra ou extra-articulaire, particulièrement adapté à une chirurgie sous arthroscopie.

L'impacteur sensiblement cylindrique d'un diamètre moyen inférieur au diamètre de la calotte de la base hémisphérique de la cheville est muni d'un embout étagé pouvant pénétrer à force dans le chambrage axial prévu à cet effet dans la base de la cheville, jusqu'à ce que celle-ci arrive en butée sur un épaulement de section droite arrondie dont le concavité vient épouser la forme hémisphérique de la base de la cheville.

L'impacteur ainsi muni de la cheville artificielle constitue un ensemble rigide particulièrement facile à manipuler pour l'opérateur.

L'invention sera mieux comprise par la description qui suit d'un exemple particulier de cheville artificielle et de l'impacteur associé, donnée à titre d'exemple non limitatif en référence aux dessins dans lesquels

La figure 1 représente en élévation la cheville suivant l'invention comportant une 1/2 vue en coupe droite passant par l'axe de la cheville et une 1/2 vue de la cheville telle qu'elle se présente extérieurement.

La figure 2 représente le positionnement de la cheville artificielle dans le tunnel osseux de l'articulation recevant l'implant, venant bloquer par coincement le ligament qui se trouve alors éloigné du bord tranchant du tunnel, dans l'effort d'extension qu'il subit normalement, en s'enroulant sur la calotte hémisphérique de la cheville.

La figure 3 est une représentation en élévation de l'ensemble cheville/impacteur suivant deux demi-vues dont l'une est une 1/2 coupe droite et l'autre une 1/2 vue extérieure de l'ensemble monté.

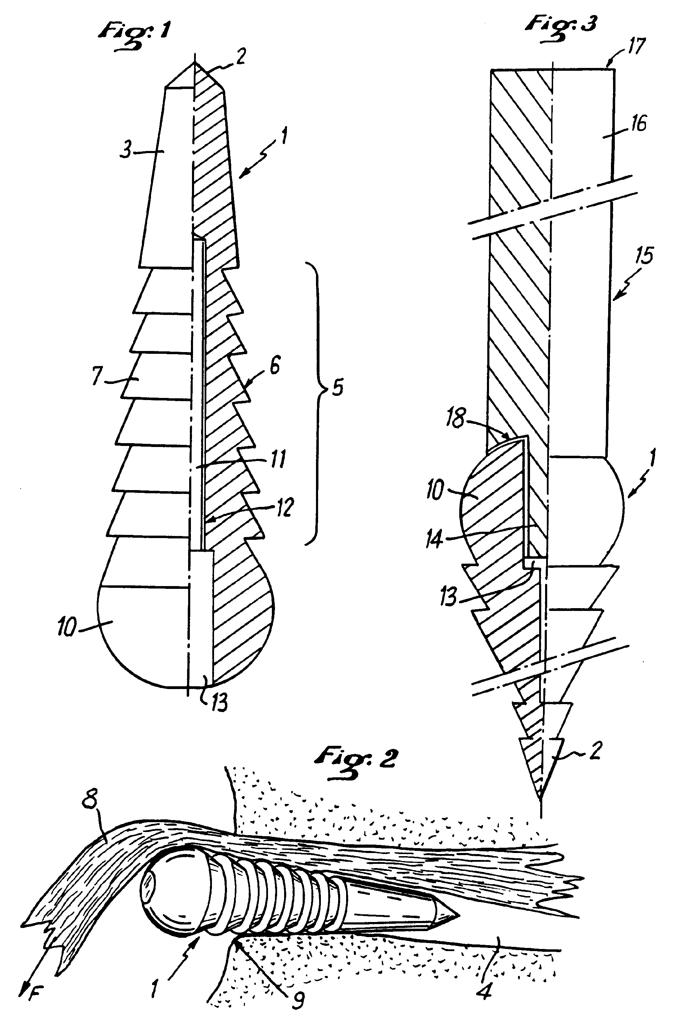

Conformément aux figures 1 et 2, la cheville 1 de forme globalement conique, réalisée en polyéthylène de haute densité, comprend extérieurement trois parties essentielles.

Au sommet 2 de la cheville 1, une première partie constituée d'un cône lisse 3, vient faciliter de manière connue le positionnement et l'introduction de la cheville 1 dans le tunnel osseux 4, pratiqué par l'opérateur dans une zone comprise entre "12 heures" et "3 heures" dans le condyle externe de l'articulation du genou ; succédant au cône lisse 3 d'entrée, une partie crantée 5 vient directement de moulage de telle manière que chaque cran 6 n'ait aucune partie débordante de l'enveloppe conique de la cheville 1 ; chaque cran 6 est constitué d'une collerette 7 sensiblement tronconique dont la petite base est dirigée vers le sommet 2 de la cheville 1, déterminant, par empilage de plusieurs collerettes 7, un système anti-retour de la cheville 1 lorsqu'elle est introduite dans le tunnel 4.

Cette zone crantée 5, sans débordement de l'enveloppe conique de la cheville 1, permet une introduction plus aisée et plus régulière, et donc un meilleur positionnement de ladite cheville 1 en vue d'éviter tout contact direct du ligament 8 bloqué par la cheville 1 dans le tunnel osseux 4, avec le bord tranchant 9 dudit tunnel 4, lors des mouvements d'extension de la jambe schématisés par F sur la figure 2. C'est pourquoi, la cheville 1 suivant une caractéristique principale de l'invention est munie d'une calotte 10 hémisphérique constituant le base de la cheville 1.

Etant observés les dimensions particulièrement réduites de ce type de cheville (comprises de préférence entre 20 et 25 mm pour la hauteur et entre 5 et 6 mm pour le diamètre de sa base) ainsi que l'affaiblissement de la cheville 1 résultant de la zone crantée 5, on a prévu de disposer un insert métallique 11 s'étendant dans l'axe de la cheville 1 depuis le premier cran 7 touchant la base 10 de la cheville 1 jusqu'à une altitude supérieure au dernier cran 7, solidaire du cône d'entrée 3 ; l'insert 10, réalisé au moyen d'un fil en acier inox d'un diamètre de l'ordre de 1 mm, est introduit à force dans un canal 12 prévu axialement à cet effet, incrusté dans le cône 3 formant le sommet de la cheville ; l'insert 10, tend à éviter les ruptures qui pourraient s'amorcer à chacune des strates définies par les collerettes 7.

Il est intéressant d'observer que l'insert 10 (qui peut d'ailleurs être positionné dans l'axe de la cheville 1 au moment de son moulage) constitue un élément radio-opaque, procurant, de manière avantageuse, une solution de repérage instantanée de la cheville 1 qu'elle soit en place dans l'articulation ou n'importe où, en cas d'arrachement accidentel du ligament 8.

La cheville 1 conforme à l'invention comporte dans la partie hémisphérique 10 de sa base un chambrage 13 destiné à recevoir l'embout 14 de l'impacteur 15 conformément à la représentation en figure 3. Ce chambrage 13 est de dimensions telles que l'embout 14 y pénètre à force pour solidariser l'impacteur 15 à la cheville 1, l'impacteur 15, réalisé en métal inoxydable, est une simple tige 16 sensiblement cylindrique de diamètre moyen inférieur au diamètre de la calotte 10 et de longueur pouvant varier en fonction de l'opération à exécuter.

A sommet 17 de la tige 16 sont aménagés de manière tout-à-fait classique une zone de préhension pour l'opérateur et une zone de frappe pour l'enfoncement de la cheville 1 dans son tunnel 4. A l'opposé du sommet 17 de la tige 16 l'embout 14 venant s'ajuster dans le chambrage 13 s'étend axialement depuis un épaulement 18 de section droite arrondie sur une longueur inférieure à la hauteur de chambrage 13 pour assurer l'épaulement 18 en appui positif sur la base 10 de la cheville 1 en épousant totalement sa forme hémisphérique de manière à concentrer les efforts longitudinaux transférés à la cheville 1.

Naturellement lorsque la cheville artificielle 1 est positionnée dans le tunnel osseux 4 il suffit de dégager l'impacteur 15 en le tirant hors du chambrage 13 de la cheville 1 maintenue solidement par son système anti-retour.

La cheville 1 ainsi décrite est particulièrement adaptée à la fixation de ligaments artificiels par voie andoarticulaire procurant un blocage de haute sécurité immédiat autorisant une reprise précoce d'activités sportives par exemple.

**Revendications**

1. Ensemble formé d'une cheville artificielle conique pour la fixation intraosseuse des prothèses ligamentaires et renforts ligamentaires disposée de façon rigide au bout d'un impacteur rendant l'ensemble particulièrement adapté à la chirurgie arthroscopique, ladite cheville (1) comportant extérieurement en partie inférieure de sa surface latérale une zone crantée (5) formant des éléments anti-retour (6) dirigés vers la base (10) de la cheville (1) caractérisée en ce que ladite base (10) est constituée d'une calotte sensiblement hémisphérique et qu'un insert (11) de renforcement de diamètre adéquat est disposé intérieurement dans un puits (12) s'étendant longitudinalement dans l'axe de la cheville (1) depuis le chambrage de fixation (13) de l'impacteur (15) prévu à la base (10) de la cheville (1) sur une longueur supérieure à la zone crantée (5).

2. Cheville conique suivant la revendication 1, caractérisée en ce que les éléments anti-retour (6) sont obtenus par enlèvement de matière à partir de la cheville (1) conique et lisse, ne procurant ainsi aucun débordement desdits éléments (6) en dehors de l'enveloppe conique de la cheville (1) assurant une régularité de pénétration et donc un meilleur positionnement de la cheville (1) sur la prothèse ligamentaire (8) qu'elle vient bloquer dans le tunnel osseux (4).

3. Cheville conique suivant l'une ou l'autre des revendications précédentes, caractérisée en ce que l'insert (11) est réalisé en métal radio-opaque, de préférence en acier inoxydable.

4. Cheville conique suivant l'une quelconque des revendications précédentes, caractérisée en ce que le chambrage de fixation (13) de l'impacteur (15) est cylindrique de diamètre identique au diamètre de l'embout lisse (14) dudit impacteur (15) qui peut ainsi être disposé à force dans le chambrage (13) sur une hauteur suffisante pour assurer une rigidité à l'ensemble cheville/impacteur (1/15).

5. Impacteur pour le positionnement et l'introduction de la cheville conique selon l'une quelconque des revendications précédentes, caractérisé en ce qu'il est de forme sensiblement cylindrique, de diamètre légèrement inférieur au diamètre de la base (10) de la cheville (1) et qu'il comporte en son extrémité dont est issu l'embout lisse (14) de fixation de la cheville (1), un épaulement (18) de forme concave homothétique de la base (10) de la cheville (1) sur lequel ledit impacteur (15) vient reposer, de manière à constituer un ensemble rigide particulièrement adapté à la chirurgie sous arthroscopie.

**Patentansprüche**

1. Vorrichtung mit einem künstlichen konischen Dübel zur intraossealen Fixierung von Bänderprothesen und Bänderverstärkungen, der am Vorderende eines Einschlaginstrumentes befestigt ist, so daß die Vorrichtung sich besonders für die arthroskopische Chirurgie eignet, wobei der Dübel (1) außen in einem unteren Teil seiner Seitenfläche eine gerippte Zone (5) aufweist, welche zur Basis (10) des Dübels (1) hin gerichtete Widerhakenelemente (6) bildet, **dadurch gekennzeichnet**, daß die Basis (10) aus einer im wesentlichen halbkugelförmigen Kalotte besteht und daß ein Verstärkungseinsatz (11) geeigneten Durchmessers innen in einem Schacht (12) angeordnet ist, der von einer Einsenkung (13) zur Befestigung des Einschlaginstrumentes (15), die an der Basis (10) des Dübels (1) in einem Stück über der gerippten Zone (5) vorgesehen ist, longitudinal in der Achse des Dübels (1) verläuft.

2. Konischer Dübel nach Anspruch 1, **dadurch gekennzeichnet**, daß die Widerhakenelemente (6) ausgehend von einem konischen und glatten Dübel (1) durch Entfernung von Material erhalten sind, keinen Überstand dieser Elemente (6) über den konischen Umriß des Dübels (1) ergeben und ein regelmäßiges Eindringen und damit eine bessere Positionierung des Dübels (1) auf der Bänderprothese (8), welche in einem Knochenkanal (4) zu sichern ist, gewährleisten.

3. Konischer Dübel nach einem der vorhergehenden Ansprüche, **dadurch gekennzeichnet**, daß der Einsatz (11) aus radio-opakem Metall besteht, vorzugsweise aus nichtrostendem Stahl.

4. Konischer Dübel nach einem der vorhergehenden Ansprüche, **dadurch gekennzeichnet**, daß die Ansenkung (13) zur Befestigung des Einschlaginstrumentes (15) zylindrisch ist und einen Durchmesser hat, der gleich dem Durchemesser eines glatten Ansatzstückes (5) des Einschlaginstrumentes (15) ist, so daß dieses mit Zwang über eine ausreichende Strecke in die Ansenkung (13) eingeführt werden kann, um eine genügende Festigkeit der Dübel/Einschlaginstrument-Vorrichtung (1/15) zu gewährleisten.

5. Einschlaginstrument zum Positionieren und einführen eines konischen Dübels gemäß einem der vorhergehenden Ansprüche, **dadurch gekennzeichnet**, daß es im wesentlichen zylindrisch ist und einen Durchmesser hat, der geringfügig kleiner ist als der Durchmesser der Basis (10) des Dübels (1), und daß es an seinem Ende, an dem das glatte Ansatzstück (14) zur Befestigung des Dübels (1) ansetzt, eine Schulter (18) mit einer konkaven und zur Basis (10) des Dübels (1), an der das Einschlaggerät (15) anliegt, komplementären Form aufweist, so daß sich eine starre Vorrichtung ergibt, die sich besonders für die arthroskopische Chirurgie eignet.

**Claims**

1. Set composed of a coniform artificial pin for the intraosseous fixing of ligamentary protheses and ligamentary reinforcing pieces rigidly arranged at the end of an impaction instrument, making the whole particulary adapted to arthroscopic surgery, the said pin (1) having on the outside, on the lower part of its lateral surface, a notched area (5) forming reserve-lock elements (6) directed towards the base (10) of the pin (1), characterized in that the said base (10) is composed of an approximately hemispherical cap and a reinforcing insert (11) of a suitable diameter is placed inside, in a well (12) stretching longitudinally along the axis of the pin (1) from the fixing recess (13) of the impaction instrument (15) provided at the base (10) of the pin (1) over a greater length than that of the notched area (5).

2. Coniform pin in accordance with claim 1, characterized in that the reverse-lock elements (6) are obtained by removing matter from the smooth coniform pin (1), thus procuring no overlapping of the said elements (6) outside the cone shaped envelop of the pin (1) ensuring a regular penetration and therefore a better positioning of the pin (1) on the ligamentary prothesis (8) it blocks in the bone tunnel (4).

3. Coniform pin in accordance with either of the previous claims, characterized in that the insert (11) is made of radio-opaque metal, preferably stainless steel.

4. Coniform pin in accordance with one of the previous claims, characterized in that the fixing recess (13) of the impaction instrument (15) is cylindrical with a diameter identical to the diameter of the smooth end (14) of the said impaction instrument (15) which can thus be forced into the recess (13) over a length sufficient enough to ensure that the pin/impaction instrument set (1/15) is rigid.

5. Impaction instrument for the positioning and introduction of the coniform pin in accordance with one of the previous claims, characterized in that it is approximately cylindrical in shape, with a diameter slightly smaller than the diameter of the base (10) of the pin (1) and in that it includes at its end where the smooth end piece for fixing the pin (1) is to be found, a homothetic concave shaped shoulder (18) of the base (10) of the pin (1) on which the said impaction instrument (15) rests, so as to form a rigid set particulary adapted to arthroscopic surgery.

Fig. 1

Fig. 3

Fig. 2